# EUROPEAN PATENT SPECIFICATION

(11) **EP 2 041 292 B1**
(45) Date of publication and mention of the grant of the patent: **19.10.2011**
(21) Application number: 06757817.9
(22) Date of filing: 03.07.2006
(51) Int. Cl.: C12P 3/00, C12P 5/02, C12P 7/10

(54) **Pre-treatment of biomass**
Vorbehandlung von Biomasse
Prétraitement de Biomasse

(43) Date of publication of application: 01.04.2009
(73) Proprietor: Hendriks, Antonius Theodorus Wilhelminus Maria, 6604 KH Wijchen (NL)
(72) Inventor: Hendriks, Antonius Theodorus Wilhelminus Maria, 6604 KH Wijchen (NL)
(74) Representative: Vogels, Leonard Johan Paul
(86) International application number: PCT/NL2006/000327
(87) International publication number: WO 2008/004853

(56) References cited:
- WO-A-2006/110891
- FR-A1- 2 518 573
- US-A- 4 644 060
- US-A- 5 705 369
- US-A- 5 865 898
- US-A1- 2002 102 673
- BASAGLIA M ET AL: "ENHANCED DEGRADATION OF AMMONIUM-PRETREATED WHEAT STRAW BY LIGNOCELLULOLYTIC STREPTOMYCES SPP" CANADIAN JOURNAL OF MICROBIOLOGY, OTTAWA, CA, vol. 38, no. 10, 1992, pages 1022-1025, XP008067511 ISSN: 0008-4166
- KURAKAKE M ET AL: "PRETREATMENT WITH AMMONIA WATER FOR ENZYMATIC HYDROLYSIS OF CORN HUSK, BAGASSE, AND SWITCHGRASS" APPLIED BIOCHEMISTRY AND BIOTECHNOLOGY, CLIFTON, NJ, US, vol. 90, no. 3, March 2001 (2001-03), pages 251-259, XP008067510 ISSN: 0273-2289

## Description

The present invention relates to a method of pretreatment of biomass, in order to improve the conversion of said biomass. . Further, the present invention relates to a method of converting pretreated biomass into further products.

Lignin is the world's most abundant non-carbohydrate biomaterial. It is a three dimensional macromolecule of enormously high molecular weight. Since its units are extensively cross-linked, it is difficult to define an individual molecule. Lignin provides strength by binding cellulose fibrils together. Being hydrophobic in nature, it prevents water loss from the vascular system and, being highly resistant to enzymatic degradation, it protects plants from insects and microbial attack. Lignin protects the plants also from microbial attack by binding enzymes.

To enhance susceptibility to e.g. subsequent enzymatic hydrolysis, lignocellulose pre-treatment is an essential requirement. The heterogeneous enzymatic degradation of lignocellulosics is primarily governed by its structural features because (1) cellulose possesses a highly resistant crystalline structure, (2) the lignin surrounding the cellulose forms a physical barrier, (3) the lignin binds proteins and (4) limits the sites available for enzymatic attack. An ideal pre-treatment, therefore, would increase the accessible surface area for the enzymes, reduce lignin content and/or inactivate the protein binding capacity of lignin, and have a concomitant reduction in crystallinity.

In the state of the art such pre-treatment methods are known. For instance, US patent 5,865,898 describes methods of biomass pre-treatment, wherein a lignocellulose containing biomass is pre-treated by adding calcium hydroxide, water and an oxidizing agent to the biomass to form a mixture, in order to oxidize the biomass without degrading the lignocellulose.

The mixture is heated to 40 °C to 150 °C for a period of between 1 hour and 36 hours. Per gram dry biomass 6 to 19 grams of water is added. The biomass obtained is meant for feedstock for animals, wherein the use of calcium hydroxide does not seem to alter the digestibility. Furthermore, it is aimed at keeping the lignocellulose intact.

A disadvantage of this prior art method is that a relatively high pH is present, typically a pH of 9 or higher. Furthermore, the consumption of Ca²⁺ is relatively high, through binding to the biomass. Another drawback of said method is that the Ca²⁺ needs to be removed, thereby forming salts, before further processing of the pre-treated biomass, as the Ca²⁺ may negatively interfere with further processes, thereby deteriorating the yield and quality of the products formed. For instance, the salt concentration may be harmful to micro-organisms or enzymes, or the salt may be present at unwanted locations, such as in pipes in an apparatus, thereby clocking the pipe and preventing proper heat dissipation. Typically the pre-treated biomass will need to be neutralized, as the further processing may require a neutral or acid environment, thereby forming abundant amounts of salts. Therefore the use of Ca(OH)₂ may be useful in processes for producing feedstock, but it has serious drawbacks in many other processes, e.g. those producing biofuel.

Incidentally a process is known that uses liquid ammonia at high pressure in order to treat a lignocellulose containing biomass (Dale et al. in Bioresource Technology 56 (1996), p. 111-116). However, the liquid ammonia and high pressure put serious constraints to the equipment used and involve serious risks in terms of safety and environment. Furthermore, the treated biomass needs to undergo subsequent processing, before further conversion is feasible. Also the process is carried out without significant amounts of water being present.

Further, EP 0 415 959 discloses a process, wherein biomass is treated with NaOH and oxygen at a relatively high pH of 10.5-12.5.

US 5 705 369 discloses the pretreatment of lignocellulosic substrates with weak acids/bases, e.g. urea, guanidine, amino compounds, and with ammonia. The pretreatment conditions are less severe conditions and at less extreme pH compared to the prior art. The pretreated material is optionally further pretreated with an oxidizing agent and the material is subsequently converted into e.g. ethanol by anaerobic fermentation. Optionally, the pretreatment agent is also recycled.

BASAGLIA M ET AL: CANADIAN JOURNAL OF MICROBIOLOGY, vol. 38, no. 10, 1992, pages 1022-1025 disclose the pretreatment of wheat straw with NH4HCO3 for fermentation to enhance access of degrading enzymes to cellulose.

FR 2 518 573 discloses the pretreatment of lignocellulosic materials with ethanolamine.

KURAKAKE M ET AL: APPLIED BIOCHEMISTRY AND BIOTECHNOLOGY, vol. 90, no. 3, March 2001, pages 251-259 disclose the pretreatment of bagasse etc. with diluted ammonia solution before saccharification.

WO 2006/110891 discloses the pretreatment of lignocellulose biomass with dilute ammonia at a pH in the range of 7-9 and the subsequent fermentation of this biomass to ethanol. The recycling of the pretreatment agent is also disclosed.

US 2002/0102673 discloses a method for the production of methane from biomass by anaerobic digestion, wherein the biomass is pretreated by a thermo-chemical method using ammonia at low concentrations.

A first object of the present invention is to provide a simple method of pre-treating a lignocellulose containing biomass, which forms a biomass that can be processed further relatively easy.

A further object is to provide a method that allows for further processing of the pre-treated biomass, whereby the yield and quality of the products further formed are improved.

An other object is to provide a method that makes use of readily available chemicals and apparatus.

A next object is to provide a method that does not put too much burden on the apparatus used.

It is also an object of the present invention to improve the effectiveness of materials used in further processing, e.g. in terms of amount of chemicals and/or active substance necessary per unit of biomass and/or per unit of product formed.

Surprisingly, such a method has now been discovered by the present inventor. The present invention solves the above mentioned problems and improves existing methods by forming a mixture of the biomass, water and a suited largely recoverable nucleophilic composition in a sufficient amount,
b) reacting the nucleophilic composition with the biomass, wherein reacting takes place at a pH in the range of 4-9 *and wherein the reaction takes place under flushing with air,*
wherein the mixture contains between *0,04* to *2,5* mol of nucleophilic composition per litre mixture of biomass, wherein the nucleophilic composition comprises a compound selected from the group consisting of NH₃, NH₄⁺-salts, urea, amides, and amino-acids, and mixtures thereof, and wherein the pre-treated biomass is subsequently converted by anaerobic digestion.

The present invention is a simple method of pre-treating a lignocellulose containing biomass, which forms a biomass that can be processed further relatively easy, whereby the yield and quality of the products further formed are improved. Typically, no cleaning steps or purification steps are required after the pre-treatment. It is expected that no or less lignine is dissolved during the present process, due to the "soft" process conditions, which is favourable for subsequent processing. The process can typically be followed directly by subsequent steps, such as a conversion into ethanol, hydrogen or methane. Also, the present invention preserves valuable compounds, such as sugars, e.g. C5 and C6 sugars.

Further, the present method makes use of readily available chemicals and apparatus, and it does not put too much burden on the apparatus used, for instance the reactor used can be much smaller, less complex, safer and less energy intensive.

A next advantage is that the present invention improves the effectiveness of materials used in further processing, e.g. in terms of amount of chemicals and/or active substance necessary per unit of biomass and/or per unit of product formed.

One of the further advantages is that it is believed that the protein binding capacity of the lignocellulose containing biomass decreases. This decrease of bonding improves the effectiveness of optionally used protein containing compounds, such as enzymes, in subsequent steps. The binding of protein is currently one of the major drawbacks in subsequent steps, as it clearly greatly reduces the yield and quality of the products formed, and/or requires larger amounts of enzymes in order to obtain acceptable yields, which latter is economically not favourable.

Biomass can be classified in three main categories: sugar-, starch- and cellulose-containing plants. Sugar-containing plants (e.g. sweet sorghum, sugarcane) and starch-containing plants (e.g. corn, rice, wheat, sweet potatoes) are primarily used as food sources. Cellulose-containing plants and waste products (e.g. grasses, wood, bagasse, and straws) are the most abundant forms of biomass. Although they are not easily converted to useful products, a well engineered process to convert them to feedstock may potentially be economical since the costs of feedstock are much less than those of sugar- and starch-containing biomass.

The lignocellulose-containing biomass can be selected from a broad range of biological material, such as material specifically grown for its biomass, such as wood, or material that is a by product of agriculture, such as waste due to harvesting. Preferably the lignocellulose-containing biomass is selected from the group consisting of grass, wood, bagasse, straw, paper, sawdust, cotton, corn, plant material, and combinations thereof. Typically cellulose-containing materials are generally referred to as lignocellulosics because they contain cellulose (40% -60%), hemicellulose (20% -40%) and lignin (10% -25%). Non-woody biomass generally contains less than about 15-20% lignin. The present method is expected to be capable of handling a biomass with a large amount of lignocellulose, such as 35% as is the case in e.g. coconut shells.

It is noted that all percentages given are taken relative to the total amount of biomass, unless stated otherwise.

Advantageously the biomass does in general not require any activation of the biomass, such as treatment with propylene oxide or hydroxypropylate, whereas, on the contrary, several state of the art processes do.

A further advantage is that the nucleophile can be largely recovered, typically 50% or more, preferably 70% or more, more preferably 90% of the nucleophile can be recovered, typically without too much burden, whereas in state of the art methods a nucleophile (if used at all and/or as such) forms e.g. salts (see above), or binds more or less irreversible to the biomass.

In cases where a nucleophile, or a compound formed thereof, is volatile, the reaction preferably takes place in a closed atmosphere, such as a closed reactor, in order to prevent evaporation of the volatiles.

The reacting takes place at a pH in the range of 4-9, more preferably at a pH in the range of 4.8-8.5, even more preferably in the range of 6-7.5. It is noted that the pH may be used to govern the present method, as the pH may influence the equilibrium of optional reactions of the nucleophilic composition with acid or base, as the case may be. If the pH becomes too high a neutralisation step may become necessary before subsequent processing, whereas at low pH the amount of nucleophile expected to be bonded to the biomass may be too low. Due to the fact that the process takes place at more or less neutral pH, only a low concentration of acid or base, as the case may be, is present. This latter phenomenon is very advantageous for further processing, as no or almost no extra steps are required, such as neutralization, in order to make the pretreated biomass ready for further processing.

Preferably the reacting takes place at a temperature in the range of 40 to 170 °C, more preferably in the range of 80 to 160 °C, even more preferably in the range of 100 to 140 °C. It is important to note that a higher temperature may be accompanied by a higher pressure, if the nucleophilic composition, or a compound formed thereof, is or can become volatile. If the temperature is too low, the reaction between nucleophile and lignine will be too slow. Also the softening of the lignocellulose containing material will not take place sufficiently. It is important that the temperature is high enough, typically equal or above the glass transition temperature of the lignocellulose containing biomass, in order to keep the pre-treated biomass swollen long enough after the pre-treatment. If the temperature is too high the lignine may dissolve into the solution, which is detrimental for subsequent processing.

Preferably the reacting takes place during a time in the range from 5 minutes to 36 hours, more preferably in the range from 10 minutes to 24 hours, even more preferably in the range from 15 minutes to 6 hours. If the reaction time is too short, the reaction may not have been taken place sufficiently, whereas longer reaction times do not improve the yield and/or quality of the optional subsequent process, aimed at conversion into e.g. ethanol or methane.

The reacting takes place at a nucleophilic concentration in the range of 0.04 to 2.5 mol nucleophile/l, more preferably at a concentration in the range of 0.1 to 1 mol nucleophile/l. If the concentration is too low, no or only a small improvement is observed. If the concentration is too high, no further improvement is observed, whereas in the latter case a burden is placed on safety and environment. It is a clear advantage of the present method that relatively low amounts of nucleophile can be used, which are still very effective in terms of yield and quality (see above).

Preferably the reacting takes place at a pressure in the range of from 0,1 to 35 bar, more preferably in the range of from 1 to 5 bar. If the pressure is too low too much volatiles may escape from the solution (see above), whereas too high pressures place an extra burden on the apparatus used.

All molecules or ions with a free pair of electrons can act as nucleophiles, although negative ions (anions) are more potent than neutral reagents. However, preferably the nucleophilic composition comprises a compound that reacts specific with the lignin. Without wishing to be bound by theory, it is believed that it is important that the nucleophile binds to lignine, thereby it is likely that the lignine surface becomes more hydrophile, in order to improve yield and quality of subsequent processing. It is believed that an appropriate nucleophile improves the swelling and reduces binding of protein to lignine. In the invention such a compound is selected from the group consisting of NH₃, NH₄⁺-salts, urea, amides and amino-acids and mixtures thereof. Such a specifically binding nucleophile has as is expected the advantage that also the softening of the lignocellulose containing material will take place sufficiently. Yet a further advantage as the case may be is that an amine or ammonia containing compound used is typically not very harmful to organisms optionally used in further processing.

Preferably between about 5 to about 20 grams of water per gram of dry biomass are added to the mixture. The main item here is of practical nature, namely for processing the biomass it may be necessary that the mixture thereof can be pumped from one location to the other.

Preferably the pre-treatment does not decompose said biomass, as decomposition may make the nature and measures to be taken while further processing more difficult.

Preferably the mixture is dry mixed before pretreatment. Preferably the mixture is continuously mixed during pre-treatment. A well mixed mixture may speed up the pre-treatment as well as subsequent processing.

In order to improve the method even further an oxidizing agent may be added to the mixture. Such an oxidizing agent preferably contains oxygen, such as gasses as oxygen, air, or peroxides, such as hydrogen peroxide. If the oxidizing agent is a gas it is preferably added to the mixture at a pressure of between about 0,1 to about 50 bar, more preferably between 1 and 35 bar. The use of an oxidizing agent can further improve the yield and quality of the products formed. It is currently believed that, amongst others, an oxidizing agent, such as oxygen, increases the binding of the nucleophile to the lignocellulose containing biomass, which is advantageous in subsequent steps (see above). Furthermore, it implies that a further advantage of the present process is that it can be carried out under ambient conditions, that is, in this respect, no special care needs to be taken to remove oxidizing agents, such as e.g. oxygen or air, which clearly advantageously limits the requirements for equipment and materials used.

In order to reduce possible side reactions, likely to occur due to the presence of for instance metal ions, an gelating agent may be added to the mixture. Such gelating agents preferably are chosen from the group consisting of silicates, ethylene diamine, EDTA and porphine. In view of environmental restrictions the gelating agent is preferably non-toxic. It is noted that on the other hand gelating mixtures may be detrimental to catalyst action of e.g. metal ions.

In a second aspect the present invention relates to a method wherein the biomass pretreated by the method of the invention is subsequently converted by a process selected from the group of acid hydrolysis, enzymatic action, fermentation, aerobic digestion, anaerobic digestion or a combination thereof.

Cellulose, a glucose polymer, can be hydrolyzed to glucose using acid, enzymes or microbes. Glucose can serve as a feedstock for fuel alcohol and single-cell protein production. Microbial hydrolysis produces cellular biomass (single-cell protein) and metabolic waste products such as organic acids. Acid hydrolysis, although simple, produces many undesirable degradation products. Enzymatic hydrolysis is the cleanest and most preferred approach. However, production of enzymes, mainly cellulase and cellobiase, can be an expensive step. Also, anaerobic fermentation using rumen micro organisms can produce low molecular weight volatile fatty acids.

The pre-treated biomass is converted by hydrolysis such as acid hydrolysis, enzymatic action, fermentation, or a combination of digestion methods. The digested biomass comprises material which are useful products such as alcohols, acids such as organic acids, sugars, ketones, starches, fatty acids, or combinations thereof. These products can be made into feed stocks such as chemical feed stocks, fuels, and other useful products. Due to the relatively gentle pre-treatment conditions, the useful products are obtained in higher quantities and are of a higher quality, e.g. more pure, than products obtained after other pre-treatment methods. The maximum amount of material is converted into useful product with as little waste as possible. Further, no toxins or harmful chemicals are introduced into the biomass therefore none need to be removed or even tested for in the final product.

It is clear that, after pre-treatment of the biomass, the biomass can be converted into desired products. Many techniques or process types are at present available in order to accomplish such a conversion. The specific conditions of the conversion can easily determined with routine experiments. For instance, if conversion by enzymatic reaction is desired, typically less than 5 IU enzyme/g biomass are added to the biomass. Therefore it is an important advantage of the present pre-treatment method, in that significantly less enzyme per unit biomass is needed and at the same time yield and quality remain high, or, equivalently, at similar enzyme concentration per unit biomass much higher yield and quality are achieved. The latter advantage is also present for anaerobic digestion, specifically for the enzymatic part of the digestion.

Preferably a biofuel is formed. With present state of the art processes the biofuel may be selected form the group of ethanol, hydrogen or methane.

The present disclosure relates also to a method for recovering nucleophilic composition from a biomass pretreatment process comprising the steps of:
a) pre-treating the biomass according to the invention,
b) recovering the nucleophilic composition.

After pre-treatment the nucleophilic composition can easily be recovered by standard means. Thereafter it can be fed back to a reaction system in a closed loop. If the nucleophilic composition comprises e.g. ammonium, this compound can also be released upon subsequent processing. The thus released compound can again be fed to a reaction system. Thereby the consumption of chemicals is significantly reduced.

The present invention is further elucidated by the following example, which by no means is meant to limit the scope of the present invention.

### Example 1

About 0,24 gram straw is dissolved in 150 ml water with an ammonium concentration of about 4000 mg/l NH₄-N (added as ammonium carbonate ((NH₄)₂CO₃). This mixture is transferred into serum bottles of about 250 ml and closed with a rubber cover with an aluminium cap on top thereof. Three serum bottles are flushed with nitrogen gas (oxygen free atmosphere) and three serum bottles are flushed with air, thereby introducing oxygen. The pH was about 8,3.

These six serum bottles are subsequently transferred to an auto-clave and heated to about 120 ° C during two hours. During this pre-treatment one of the three bottles that was flushed with air was burst.

After the pre-treatment the solid and the liquid fraction were separated. The solid was transferred to a jar of approximately 600 ml. Also 250 ml tap water, 1.5 ml macro nutrients, 0.15 ml micro nutrients, a Sørensen buffer (20 mM in the jar) (phosphate buffer to obtain a pH around 7), and approximately 7 grams of AVIKO granules were added to each bottle. An oxi-top was used to measure the pressure difference, and the gas composition was analysed to calculate the amount of CH₄. After putting everything into the bottle the headspace of the bottle was flushed with N2 before closing it with the oxi-top. After that the bottles were placed at a constant temperature of 35 °C.

The macro nutrients stock solution when working with a Sørensen buffer comprises:

| | | |
|---|---|---|
| NH₄Cl | 170 | g/L |
| CaCl₂.2H₂O | 8 | g/L |
| MgSO₄.7H₂O | 9 | g/L |

The trace elements stock solution used comprises:

| | | |
|---|---|---|
| FeCl₃.4H₂O | 2 | g/L |
| CoCl₂.6H₂O | 2 | g/L |
| MnCl₂.4H₂O | 0.5 | g/L |
| CuCl₂.₂H₂O | 30 | mg/L |
| ZnCl₂ | 50 | mg/L |
| HBO₃ | 50 | mg/L |
| (NH₄) 6Mo₇O₂.4H₂O | 90 | mg/L |
| Na₂SeO₃.5H₂O | 100 | mg/L |
| NiCl₂.6H₂O | 50 | mg/L |
| EDTA | 1 | g/L |
| HCl 36% | 1 | ml/L |
| Resazurin | 0.5 | g/L |

It was found that the compounds present in the liquid fraction, such as the solubilized fatty acids, could easily be converted into e.g. methane. No inhibiting effect was observed. It is therefore likely that no inhibiting compounds were produced during the pre-treatment. This is important, because inhibiting compounds would than also be present in the solid fraction, after pre-treatment, which inhibiting compounds could seriously jeopardise the yield and quality of the subsequently formed products, such as methane.

It may thus also be concluded that a separation form the solid fraction and the liquid fraction is not necessary, from the point of view of further processing, which is a clear advantage.

In table 1 the results are shown.

**Table 1: results.**

| | Untre | Untre | 1 air flushed | 2 air flushed | 1 | 2 | 3 | |
|---|---|---|---|---|---|---|---|---|
| | ated | ated | | | nitrogen | nitrogen | nitrogen | average |
| | 1 | 2 | | | flushed | flushed | flushed | |
| mmol | | | | | | | | |
| CH4/ gr | | | | | | | | |
| COD of | 3.75 | 3.62 | 5.66 | 10.56 | 4.73 | 6.70 | 5.89 | 6.71 |
| wheat | | | | | | | | |
| straw | | | | | | | | |
| Yield | | | | | | | | |
| compared | | | | | | | | |
| to | 102 | 98 | 154 | 286 | 128 | 182 | 160 | 182 |
| untreated | | | | | | | | |
| wheat | | | | | | | | |
| straw (%) | | | | | | | | |

| | | | | | | | | |
|---|---|---|---|---|---|---|---|---|
| COD = Chemical Oxygen Demand 1 gr COD of wheat straws = an amount of wheat straw that needs 1 gr of oxygen to decompose it to CO2 and H20. | | | | | | | | |

'Untreated 1' and 'untreated 2' are the two untreated, though otherwise commonly digested wheat straw samples, that is, without pre-treatment according to the invention.

'1 air flushed' and '2 air flushed' are the digested wheat straw samples that contained 1 bar air during the pretreatment according to the invention.

'1 nitrogen flushed','2 nitrogen flushed', and '3 nitrogen flushed' are the digested wheat straw samples that contained nitrogen gas during the pre-treatment according to the invention.

All samples were digested according to common digestion techniques, as mentioned above.

'Average' is the average yield of the pre-treated samples.

The row 'mmol CH₄/gr COD straw' gives the amount mmol CH₄ produced per gram COD wheat straw per sample.

The row 'yield compared to untreated wheat straw' gives the amount of CH₄ produced, in percentages, compared to the average amount (column 2 and 3, i.e. 3.685) of CH₄ produced by the two untreated samples.

The conclusion therefore is that the pre-treated biomass has a yield that is on average 182% compared to the untreated biomass. The pre-treatment in this example thus gives an increase of 82% in yield. It is noted that this result is obtained even without further optimisation of the process conditions. The large standard deviation, e.g. the "2 air flushed" value may even imply that much higher yields can be achieved.

## Claims

1. Method for pre-treating a lignocellulose-containing biomass, comprising the steps of:
a) forming a mixture of the biomass, water and a suited largely recoverable nucleophilic composition in a sufficient amount,
b) reacting the nucleophilic composition with the biomass, wherein reacting takes place at a pH in the range of 4-9 *and wherein the reaction takes place under flushing with air,*
wherein the mixture contains between *0,04* to *2,5* mol of nucleophilic composition per litre mixture of biomass, wherein the nucleophilic composition comprises a compound selected from the group consisting of NH₃, NH₄⁺-salts, urea, amides, and amino-acids, and mixtures thereof, and wherein the pre-treated biomass is subsequently converted by anaerobic digestion.

2. Method according to claim 1, wherein reacting takes place at a temperature in the range of 40 to 170 °C, during a time in the range from 5 minutes to 36 hours, and at a pressure in the range of from 1 bar to 35 bar.

3. Method according to claim 1, wherein the lignocellulose-containing biomass is selected from the group consisting of grass, wood, bagasse, straw, paper, plant material, and combinations thereof.

4. Method according to claim 1, wherein further an oxidizing agent is added to the mixture.

5. Method according to claim 1, wherein the oxidizing agent is a gas and is added to the mixture at a pressure of between about 0,1 to about 35 bar.

6. The method according to claim 1, wherein a biofuel is formed selected form the group of ethanol, hydrogen or methane.

## Patentansprüche

1. Verfahren zur Vorbehandlung eines Celluligninsenthaltende Biomasse, umfassend die Schritte von:
a) das Formen einer Mischung von der Biomasse, Wasser und eines geeignete meistens zurück zu gewinnen nucleophile Zusammenstellung in einer genügende Menge,
b) das Reagieren von der nucleophile Zusammenstellung mit der Biomasse, worin Reagieren Statt findet bei einer pH im Bereich von 4-9 *und Reagieren* Statt findet unter Spulen mit Luft,
worin der Mischung zwischen *0,04* bis *2,5* mol nucleophiler Zusammenstellung pro Liter Mischung Biomasse enthalt, worin der nucleophile Zusammenstellung umfasst einer Verbindung gewählt aus die Gruppe bestehend aus NH₃, NH₄⁺-Salz, Harnstoff, amides, und amino-Sauren, und Mischungen davon, und worin der vorbehandelte Biomasse danach umgesetzt wird durch anaerober Digestion.

2. Verfahren gemäß Anspruch 1, worin Reagieren Statt findet bei einer Temperatur in Bereich von 40 bis 170 °C, wahrend einer Periode im Bereich von 5 Minuten bis 36 Stunden, und bei einer Druck im Bereich von 1 bar bis 35 bar.

3. Verfahren gemäß Anspruch 1, worin der Celluligninsenthaltende Biomasse ist gewählt aus die Gruppe bestehend aus Gras, Holz, Bagasse, Stroh, Papier, Pflanz-material, und Kombinationen davon.

4. Verfahren gemäß Anspruch 1, worin weiter eines oxidierend Mittel ist zugefugt an der Mischung.

5. Verfahren gemäß Anspruch 1, worin das oxidierend Mittel eines Gas ist und ist zugefugt an der Mischung bei einer at Druck im von etwa 0,1 bis etwa 35 bar.

6. Verfahren gemäß Anspruch 1, worin eines Biobrennstoff ist geformt gewählt aus die Gruppe von Ethanol, Wasserstoff oder Methan.

## Revendications

1. Procédé de prétraitement biomasse comprenant lignocellulose, comprenant les étapes de:
a) former un mélange biomasse, eau et un composition nucléophile agréable pour la plupart recouvrable dans une quantité suffise,
b) réagir la composition nucléophile avec la biomasse, dans lequel réagir se passe a un pH dans le intervalle 4-9 et dans lequel la *réaction* se passe avec rincer avec de l'*air*,
dans lequel le mélange contient entre *0,04* et *2,5* mol de composition nucléophile per litre mélange de biomasse, dans lequel le composition nucléophile comprenant un combinaison sélecter de la groupe de sals de NH₃, NH₄⁺, urée, amides, et des acides amino, et mélanges de cela, et dans lequel le biomasse prétraitement est convertir subséquent avec digestion anaérobie.

2. Procédé selon la revendication 1, dans lequel réagir se passe a une température dans le intervalle 40 a 170 °C, durent une temps dans le intervalle de 5 minutes a 36 heures, et a une pression dans le intervalle de 1 bar a 35 bar.

3. Procédé selon la revendication 1, dans lequel le biomasse comprenant lignocellulose est sélecter de le groupe contenant herbe, bois, bagasse, paille, papier, matérielle de plante, et de cela.

4. Procédé selon la revendication 1, dans lequel de plus une agent oxydant est ajoute de la mélange.

5. Procédé selon la revendication 1, dans lequel l'agent oxydant est une gaz et est ajoute de la mélange a une pression de approximent 0,1 a approximent 35 bar.

6. Procédé selon la revendication 1, dans lequel un biocarburant est forme sélecter de le groupe de éthanol, hydrogène ou méthane.
